(19) **Europäisches Patentamt / European Patent Office / Office européen des brevets**

(11) **EP 3 813 663 B1**

(12) **FASCICULE DE BREVET EUROPEEN**

(45) Date de publication et mention
de la délivrance du brevet:
**10.04.2024 Bulletin 2024/15**

(21) Numéro de dépôt: **19790615.9**

(22) Date de dépôt: **27.06.2019**

(51) Classification Internationale des Brevets (IPC):
**A61B 5/00** *(2006.01)*

(52) Classification Coopérative des Brevets (CPC):
**A61B 5/14532; A61B 5/1118; A61B 5/4866;
A61B 5/7239; A61B 5/7275; G16H 50/30;**
A61B 5/024

(86) Numéro de dépôt international:
**PCT/FR2019/051598**

(87) Numéro de publication internationale:
**WO 2020/002848 (02.01.2020 Gazette 2020/01)**

(54) **SYSTÈME AUTOMATISÉ DE CONTRÔLE DE LA GLYCÉMIE D'UN PATIENT**

AUTOMATISCHES SYSTEM ZUR KONTROLLE DES BLUTZUCKERS EINES PATIENTEN

AUTOMATED SYSTEM FOR CONTROLLING THE BLOOD SUGAR OF A PATIENT

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **29.06.2018 FR 1856016**

(43) Date de publication de la demande:
**05.05.2021 Bulletin 2021/18**

(73) Titulaire: **Commissariat à l'Energie Atomique et
aux Energies
Alternatives
75015 Paris (FR)**

(72) Inventeurs:
• **ROMERO UGALDE, Hector-Manuel
38000 GRENOBLE (FR)**
• **DORON, Eléonore-Maeva
38054 GRENOBLE CEDEX 09 (FR)**
• **BONNET, Stéphane
38054 GRENOBLE CEDEX 09 (FR)**
• **BLANC, Romain
38054 GRENOBLE CEDEX 09 (FR)**

• **SIMON, Chantal
69373 LYON CEDEX 08 (FR)**
• **GARNOTEL, Mael
69310 PIERRE-BENITE (FR)**

(74) Mandataire: **Cabinet Beaumont
4, Place Robert Schuman
B.P. 1529
38025 Grenoble Cedex 1 (FR)**

(56) Documents cités:
FR-A1- 3 056 095        US-A1- 2010 057 043
US-A1- 2016 354 543     US-B1- 6 923 763

• ROY AND R S PARKER A: "Dynamic Modelling of Exercise Effects on Plasma Glucose and Insulin Levels", JOURNAL OF DIABETES SCIENCE AND TECHNOLOGY, DIABETES TECHNOLOGY SOCIETY, US, vol. 1, no. 3, 1 mai 2007 (2007-05-01), pages 338-347, XP002660806, ISSN: 1932-2968

**Description**

La présente demande de brevet revendique la priorité de la demande de brevet français FR18/56016.

Domaine

La présente demande concerne le domaine des systèmes automatisés de contrôle de glycémie, et vise plus particulièrement un système de contrôle adapté à prédire l'évolution future de la glycémie d'un patient.

Exposé de l'art antérieur

On a déjà proposé, par exemple dans la demande de brevet internationale N°WO2018055283 (B15018/DD16959), dans la demande de brevet internationale N°WO2018055284 (B15267/ DD17175), et dans la demande de brevet français N°1756960 (B15860/DD18587) déposée le 21 juillet 2017, des systèmes automatisés de régulation de glycémie, aussi appelés pancréas artificiels, permettant de réguler automatiquement les apports en insuline d'un patient diabétique à partir de son historique de glycémie, de son historique de prise de repas, et de son historique d'injection d'insuline.

Les systèmes de régulation décrits dans les demandes de brevet susmentionnées sont des systèmes de type MPC (de l'anglais "Model-based Prédictive Control"), aussi appelés systèmes à commande prédictive, dans lesquels la régulation de la dose d'insuline administrée tient compte d'une prédiction de l'évolution future de la glycémie du patient, réalisée à partir d'un modèle mathématique. Plus particulièrement, dans les systèmes décrits dans les demandes de brevet susmentionnées, le modèle utilisé pour prédire l'évolution future de la glycémie du patient est un modèle physiologique décrivant l'assimilation de l'insuline par le corps du patient et son impact sur la glycémie du patient.

De façon plus générale, de nombreuses applications automatisées de contrôle de glycémie utilisent des modèles physiologiques pour prédire l'évolution future de la glycémie d'un patient, et mettre en oeuvre, en tenant compte de cette prédiction, des actions visant à maintenir la glycémie du patient dans une plage souhaitée.

Les modèles physiologiques connus pour la prédiction de l'évolution future de la glycémie d'un patient présentent toutefois certaines limitations. En particulier, dans certaines conditions, il arrive que les prédictions réalisées par les modèles physiologiques connus ne soient pas fiables. Ceci peut conduire à des erreurs lors du contrôle ou lors de régulation de la glycémie du patient, ce qui peut entraîner des risques pour le patient.

Résumé

Ainsi, un mode de réalisation prévoit un système automatisé de contrôle de la glycémie d'un patient, comportant une unité de traitement et de contrôle configurée pour prédire l'évolution future de la glycémie du patient à partir d'un modèle physiologique, dans lequel le modèle physiologique comporte un système d'équations différentielles décrivant l'évolution d'une pluralité de variables d'état en fonction du temps, et dans lequel au moins l'une des équations du système prend pour entrée une variable EE(t) représentative de l'évolution, en fonction du temps, de la dépense énergétique du patient,
ladite au moins une équation étant définie comme suit :

$$\frac{dQ_2}{dt} = x_1(t) \cdot Q_1(t) - [k_{12} + x_2(t)] \cdot Q_2(t) - k_{EE}.EE(t)$$

où $Q_1$ et $Q_2$ sont des variables d'état correspondant respectivement à des masses de glucose dans des premier et deuxième compartiments, où $x_1$ et $x_2$ sont des variables d'état représentant chacune une action de l'insuline sur la cinétique du glucose, et où $k_{12}$ et $k_{EE}$ sont des paramètre du modèle physiologique (601), le terme -($k_{EE}$*EE(t)) étant représentatif d'une consommation de glucose par les muscles.

Selon un mode de réalisation, le système comprend un dispositif de mesure d'une activité physique du patient, la variable d'entrée EE(t) du modèle étant calculée par l'unité de traitement et de contrôle à partir de données de sortie PA(t) du dispositif de mesure.

Selon un mode de réalisation, le dispositif de mesure comprend un capteur de mouvements du patient.

Selon un mode de réalisation, le dispositif de mesure comprend en outre un capteur du rythme cardiaque du patient.

Selon un mode de réalisation, le capteur de mouvements fournit un signal $S_{CPM}$ représentatif de mouvements effectués par le patient, et le capteur de rythme cardiaque fournit un signal $S_{HR}$ représentatif du rythme cardiaque du patient, et la variable d'entrée EE(t) du modèle est calculée comme suit par l'unité de traitement et de contrôle :

$$EE(t) = \begin{cases} \alpha_1 * S_{HR}(t) + \beta_1, & S_{HR} \geq S1 \\ \alpha_2 * S_{LC}(t) + \beta_2, & S_{HR} < S1 \, et \, S_{LC} < S2 \\ \alpha_3 * S_{LC}(t) + \beta_3, & S_{HR} < S1 \, et \, S_{LC} \geq S2 \end{cases}$$

où le signal $S_{LC}$ est une combinaison linéaire des signaux $S_{HR}$ et $S_{CMP}$, et où les grandeurs $\alpha_1$, $\alpha_2$, $\alpha_3$, $\beta_1$, $\beta_2$, $\beta_3$, S1 et S2 sont des paramètres du système.

Selon un mode de réalisation, le modèle physiologique prend en outre pour entrées une variable i(t) représentative de l'évolution, en fonction du temps, de la quantité d'insuline injectée au patient, et une variable cho(t) représentative de l'évolution, en fonction du temps, de la quantité de glucose ingérée par le patient.

Selon un mode de réalisation, le système comporte en outre un capteur de glycémie.

Selon un mode de réalisation, l'unité de traitement et de contrôle est adaptée à mettre en oeuvre une étape de calibration automatique du modèle physiologique comprenant une étape d'estimation d'un jeu de paramètres du système d'équations différentielles par minimisation d'une grandeur m représentative de l'erreur, pendant une période d'observation passée, entre la glycémie estimée à partir du modèle physiologique et la glycémie mesurée par le capteur.

Selon un mode de réalisation, la grandeur est définie comme suit :

$$m = \frac{1}{\Delta T} \sum_{t=t_0}^{t_0+\Delta T} |G^r(t) - G(t)|^2$$

où t est une variable temps discrétisée, $t_0$ est l'instant de début de la période d'observation passée, et $t_0 + \Delta T$ est l'instant de fin de la période d'observation passée.

Selon un mode de réalisation, le système comporte en outre un dispositif d'injection d'insuline, dans lequel l'unité de traitement et de contrôle est adaptée à commander le dispositif d'injection d'insuline en tenant compte de la prédiction de l'évolution future de la glycémie du patient réalisée à partir du modèle physiologique.

Brève description des dessins

Ces caractéristiques et leurs avantages, ainsi que d'autres, seront exposés en détail dans la description suivante de modes de réalisation particuliers faite à titre non limitatif en relation avec les figures jointes parmi lesquelles :

la figure 1 représente de façon schématique, sous forme de blocs, un exemple d'un système automatisé de régulation de la glycémie d'un patient ;
la figure 2 est une représentation simplifiée d'un modèle physiologique utilisé dans le système de la figure 1 pour prédire l'évolution future de la glycémie du patient ;
la figure 3 est un diagramme représentant plus en détail un exemple de réalisation du modèle physiologique de la figure 2 ;
la figure 4 est un diagramme illustrant un exemple d'un procédé automatisé de régulation de glycémie pouvant être mis en oeuvre par le système de la figure 1 ;
la figure 5 représente de façon schématique, sous forme de blocs, un exemple d'un mode de réalisation d'un système automatisé de régulation de la glycémie d'un patient ;
la figure 6 est une représentation simplifiée d'un modèle physiologique utilisé dans le système de la figure 5 pour prédire l'évolution future de la glycémie du patient ; et
la figure 7 est un diagramme représentant plus en détail un exemple de réalisation du modèle physiologique de la figure 6.

Description détaillée

De mêmes éléments ont été désignés par de mêmes références dans les différentes figures. Par souci de clarté, seuls les éléments qui sont utiles à la compréhension des modes de réalisation décrits ont été représentés et sont détaillés. En particulier, les dispositifs de mesure de glycémie et les dispositifs d'injection d'insuline des systèmes de régulation décrits n'ont pas été détaillés, les modes de réalisation décrits étant compatibles avec tous ou la plupart des dispositifs de mesure de glycémie et d'injection d'insuline connus. De plus, la réalisation matérielle de l'unité de traitement et de contrôle des systèmes de régulation décrits n'a pas été détaillée, la réalisation d'une telle unité de traitement et de contrôle étant à la portée de l'homme du métier à partir des indications fonctionnelles de la présente description. Sauf précision contraire, les expressions "approximativement", "sensiblement", "environ" et "de l'ordre de" signifient à 10 % près, de préférence à 5 % près.

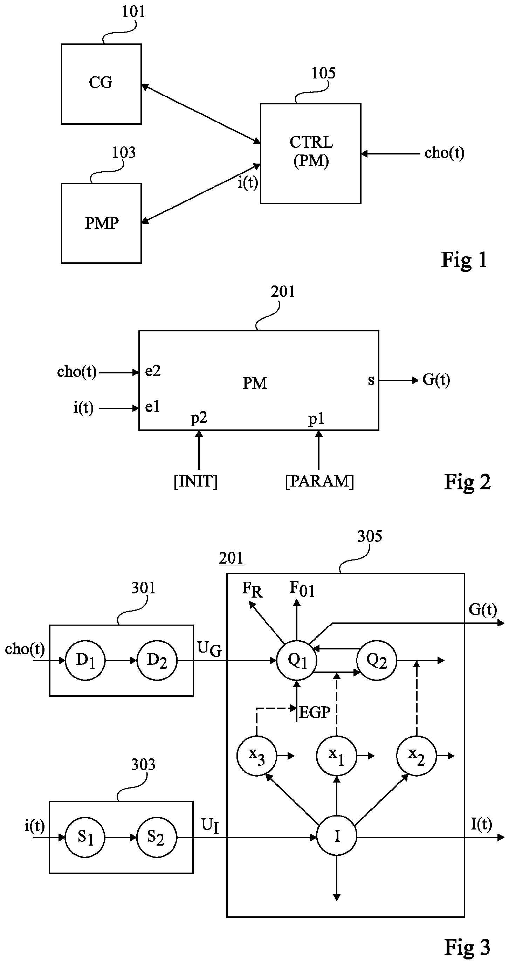

La figure 1 représente de façon schématique, sous forme de blocs, un exemple d'un système automatisé de régulation de la glycémie d'un patient du type décrit dans les demandes de brevet internationales WO2018055283 et WO2018055284 et dans la demande de brevet français N°1756960 susmentionnées.

Le système de la figure 1 comprend un capteur 101 (CG) adapté à mesurer la glycémie du patient. En fonctionnement normal, le capteur 101 peut être positionné à demeure sur ou dans le corps du patient, par exemple à hauteur de son abdomen. Le capteur 101 est par exemple un capteur de type CGM (de l'anglais "Continuous Glucose Monitoring" - surveillance continue de glycémie), c'est-à-dire un capteur adapté à mesurer en continu, par exemple de façon périodique (par exemple au moins une fois toutes les cinq minutes) la glycémie du patient. Le capteur 101 est par exemple un capteur de glycémie en sous-cutané.

Le système de la figure 1 comprend en outre un dispositif d'injection d'insuline 103 (PMP), par exemple un dispositif d'injection en sous-cutané. Le dispositif 103 est par exemple un dispositif d'injection automatique de type pompe à insuline, comportant un réservoir d'insuline relié à une aiguille d'injection implantée sous la peau du patient, la pompe pouvant être commandée électriquement pour injecter automatiquement des doses d'insuline déterminées à des instants déterminés. En fonctionnement normal, le dispositif d'injection 103 peut être positionné à demeure dans ou sur le corps du patient, par exemple au niveau de son abdomen.

Le système de la figure 1 comprend en outre une unité de traitement et de contrôle 105 (CTRL(PM)) reliée d'une part au capteur de glycémie 101, par exemple par liaison filaire ou par liaison radio (sans fil), et d'autre part au dispositif d'injection 103, par exemple par liaison filaire ou radio. En fonctionnement, l'unité de traitement et de contrôle 105 est adaptée à recevoir les données de glycémie du patient mesurées par le capteur 101, et à commander électriquement le dispositif 103 pour injecter au patient des doses d'insuline déterminées à des instants déterminés. Dans cet exemple, l'unité de traitement et de contrôle 105 est en outre adaptée à recevoir, par l'intermédiaire d'une interface utilisateur non détaillée, des données cho(t) représentatives de l'évolution, en fonction du temps, de la quantité de glucose ingérée par le patient.

L'unité de traitement et de contrôle 105 est adaptée à déterminer les doses d'insuline à injecter au patient en tenant compte notamment de l'historique de glycémie mesurée par le capteur 101, de l'historique d'insuline injectée par le dispositif 103, et de l'historique d'ingestion de glucose par le patient. Pour cela, l'unité de traitement et de contrôle 105 comprend un circuit de calcul numérique (non détaillé), comprenant par exemple un microprocesseur. L'unité de traitement et de contrôle 105 est par exemple un dispositif mobile porté par le patient tout au long de la journée et/ou de la nuit, par exemple un dispositif de type smartphone configuré pour mettre en oeuvre un procédé de régulation du type décrit ci-après.

Dans l'exemple de la figure 1, l'unité de traitement et de contrôle 105 est adaptée à déterminer la quantité d'insuline à administrer au patient en tenant compte d'une prédiction de l'évolution future de sa glycémie en fonction du temps. Plus particulièrement, l'unité de traitement et de contrôle 105 est adaptée, à partir de l'historique d'insuline injectée et de l'historique de glucose ingéré, et en se basant sur un modèle physiologique décrivant l'assimilation de l'insuline par le corps du patient et son impact sur la glycémie, à déterminer une courbe représentative de l'évolution attendue de la glycémie du patient en fonction du temps, sur une période à venir appelée période de prédiction ou horizon de prédiction, par exemple une période de 1 à 10 heures. En tenant compte de cette courbe, l'unité de traitement et de contrôle 105 détermine les doses d'insuline qu'il conviendrait d'injecter au patient pendant la période de prédiction à venir, pour que la glycémie réelle (par opposition à la glycémie estimée à partir du modèle physiologique) du patient reste dans des limites acceptables, et en particulier pour limiter les risques d'hyperglycémie ou d'hypoglycémie. Dans ce mode de fonctionnement, comme cela sera expliqué plus en détail ci-après, les données de glycémie réelle mesurées par le capteur 101 sont utilisées principalement à des fins de calibration du modèle physiologique.

La figure 2 est une représentation simplifiée d'un modèle physiologique 201 (PM) utilisé dans le système de la figure 1 pour prédire l'évolution future de la glycémie du patient. Sur la figure 2, le modèle est représenté sous la forme d'un bloc de traitement comportant :

une entrée e1 sur laquelle est appliquée un signal i(t) représentatif de l'évolution, en fonction du temps t, de la quantité d'insuline injectée au patient ;
une entrée e2 sur laquelle est appliquée un signal cho(t) représentatif de l'évolution, en fonction du temps t, de la quantité de glucose ingérée par le patient ; et
une sortie s fournissant un signal G(t) représentatif de l'évolution, en fonction du temps t, de la glycémie du patient.

Le modèle physiologique 201 est par exemple un modèle compartimental comportant, outre les variables d'entrée i(t) et cho(t) et la variable de sortie G(t), une pluralité de variables d'état correspondant à des variables physiologiques du patient, évoluant en fonction du temps. L'évolution temporelle des variables d'état et de la variable de sortie G(t) est régie par un système d'équations différentielles comportant une pluralité de paramètres représentés sur la figure 2 par un vecteur [PARAM] appliqué sur une entrée p1 du bloc 201. La réponse du modèle physiologique est en outre conditionnée par les états initiaux ou valeurs initiales affectés aux variables d'état, représentés sur la figure 2 par un vecteur [INIT] appliqué sur une entrée p2 du bloc 201.

La figure 3 est un diagramme représentant plus en détail un exemple du modèle physiologique 201 utilisé dans le système de la figure 1 pour prédire l'évolution future de la glycémie du patient. Cet exemple de modèle, connu sous le nom de modèle de Hovorka, est décrit plus en détail dans l'article intitulé "Nonlinear model prédictive control of glucose concentration in subjects with type 1 diabètes" de Roman Hovorka et al. (Physiol Meas. 2004;25:905-920), et dans l'article intitulé "Partitioning glucose distribution/transport, disposal, and endogenous production during IVGTT", de Roman Hovorka et al. (Am J Physiol Endocrinol Metab 282: E992-E1007, 2002).

Le modèle physiologique de la figure 3 comprend un premier sous-modèle bi-compartimental 301 décrivant l'effet d'une prise alimentaire de glucose sur le taux d'apparition du glucose dans le plasma sanguin. Le sous-modèle 301 prend pour entrée la quantité de glucose ingérée cho(t), par exemple en mmol/min, et fourni à sa sortie un taux $U_G$ d'absorption du glucose dans le plasma sanguin, par exemple en mmol/min. Le sous-modèle 301 comprend deux variables d'état $D_1$ et $D_2$ correspondant respectivement à des masses de glucose, par exemple en mmol, dans des premier et deuxième compartiments.

Le modèle de la figure 3 comprend en outre un deuxième sous-modèle bi-compartimental 303 décrivant l'absorption, dans le plasma sanguin, de l'insuline administrée au patient. Le sous-modèle 303 prend pour entrée la quantité d'insuline i(t) injectée au patient, par exemple en mU/min (où U désigne une unité internationale d'insuline, soit l'équivalent biologique d'environ 0,0347 mg d'insuline humaine), et fourni à sa sortie un taux $U_I$ d'absorption de l'insuline dans le plasma sanguin, par exemple en mU/min. Le sous-modèle 303 comprend deux variables d'état $S_1$ et $S_2$ correspondant respectivement à des masses d'insuline, par exemple en mmol, dans des premier et deuxième compartiments.

Le modèle de la figure 3 comprend de plus un troisième sous-modèle 305 décrivant la régulation du glucose par le corps du patient. Le sous-modèle 305 prend pour entrées les taux d'absorption $U_G$ du glucose et $U_I$ de l'insuline, et fourni à sa sortie la glycémie G(t), c'est-à-dire la concentration en glucose dans le plasma sanguin, par exemple en mmol/l. Le sous-modèle 305 comprend six variables d'état $Q_1$, $Q_2$, $x_3$, $x_1$, $x_2$, I. Les variables $Q_1$ et $Q_2$ correspondent respectivement à des masses de glucose, par exemple en mmol, dans des premier et deuxième compartiments. Les variables $x_1$, $x_2$, $x_3$ sont des variables sans unité représentant chacune des actions de l'insuline sur la cinétique du glucose. La variable I correspond à l'insulinémie, c'est-à-dire la concentration en insuline dans le plasma sanguin, par exemple en mU/l. On notera que dans l'exemple de la figure 3, on a représenté une sortie délivrant un signal I(t) représentatif de l'évolution, en fonction du temps, de la variable I. Cette sortie est toutefois optionnelle.

Le modèle de Hovorka est régi par le système d'équations suivant :

$$G(t) = \frac{Q_1(t)}{V_G} \tag{eq1}$$

$$\frac{dQ_1}{dt} = -\left[\frac{F_{01}^c}{V_G \cdot G(t)} + x_1(t)\right] \cdot Q_1(t) + k_{12}Q_2(t) - F_R + EGP_0 \cdot [1 - x_3(t)] + U_G(t) \tag{eq2}$$

$$\frac{dQ_2}{dt} = x_1(t) \cdot Q_1(t) - [k_{12} + x_2(t)] \cdot Q_2(t) \tag{eq3}$$

$$\frac{dx_1}{dt} = -k_{b1} \cdot x_1(t) + k_{a1} \cdot I(t) \tag{eq4}$$

$$\frac{dx_2}{dt} = -k_{b2} \cdot x_2(t) + k_{a2} \cdot I(t) \tag{eq5}$$

$$\frac{dx_3}{dt} = -k_{b3} \cdot x_3(t) + k_{a3} \cdot I(t) \qquad (eq6)$$

$$\frac{dS_1}{dt} = i(t) - k_a \cdot S_1(t) \qquad (eq7)$$

$$\frac{dS_2}{dt} = k_a \cdot S_1(t) - k_a \cdot S_2(t) \qquad (eq8)$$

$$\frac{dI}{dt} = \frac{k_a \cdot S_2(t)}{V_I} - k_e \cdot I(t) \qquad (eq9)$$

$$\frac{dD_1}{dt} = cho(t) - \frac{D_1(t)}{t_{max}} \qquad (eq10)$$

$$\frac{dD_2}{dt} = \frac{D_1(t)}{t_{max}} - \frac{D_2(t)}{t_{max}} \qquad (eq11)$$

$$U_G = \frac{D_2(t)}{t_{max}} \qquad (eq12)$$

Avec :

$$F_{01}^c = \frac{F_{01} \cdot G(t)}{0.85 \cdot (G(t) + 1.0)} \qquad (eq13)$$

$$F_R = \begin{cases} R(G-9) \cdot V_G & si\ G > 9 \\ 0 & sinon \end{cases} \qquad (eq14)$$

Dans ce système d'équations, les grandeurs $V_G$, $F_{01}$, $k_{12}$, $F_R$, $EGP_0$, $k_{b1}$, $k_{a1}$, $k_{b2}$, $k_{a2}$, $k_{b3}$, $k_{a3}$, $k_a$, $V_I$, $k_e$ et $t_{max}$ sont des paramètres. $V_G$ correspond au volume de distribution du glucose, par exemple en litre, $F_{01}$ correspond à un taux de transfert du glucose non insulino-dépendant, par exemple en mmol/min, $k_{12}$ correspond à une constante de taux de transfert entre les deux compartiments du sous modèle 305, par exemple en min$^{-1}$, $k_{a1}$, $k_{a2}$, $k_{a3}$ correspondent à des constantes de taux de désactivation de l'insuline, par exemple en min$^{-1}$, $F_R$ correspond à une excrétion urinaire du glucose, par exemple en mmol/min, $EGP_0$ correspond à une production endogène du glucose, par exemple en min$^{-1}$, $k_{b1}$, $k_{b2}$ et $k_{b3}$ correspondent à des constantes de taux d'activation de l'insuline, par exemple en min$^{-1}$, $k_a$ correspond à une constante de taux d'absorption de l'insuline injectée en sous-cutané, par exemple en min$^{-1}$, $V_I$ correspond au volume de distribution de l'insuline, par exemple en litre, $k_e$ correspond à un taux d'élimination de l'insuline du plasma, par exemple en min$^{-1}$, et $t_{max}$ correspond à un temps passé jusqu'au pic d'absorption du glucose ingéré par le patient, par exemple en min. Ces quinze paramètres correspondent au vecteur [PARAM] de la représentation de la figure 2. Le vecteur [INIT] comprend quant à lui dix valeurs correspondant aux valeurs initiales (à un instant $t_0$ de début d'une phase de simulation du comportement du patient à partir du modèle) affectées aux dix variables d'état $D_1$, $D_2$, $S_1$, $S_2$, $Q_1$, $Q_2$, $x_1$, $x_2$, $x_3$ et I du modèle.

Parmi les paramètres du vecteur [PARAM], certains peuvent être considérés comme constants pour un patient donné. Il s'agit par exemple des paramètres $k_{12}$, $k_{a1}$, $k_{a2}$, $k_{a3}$, $k_a$, $k_e$, $V_I$, $V_G$ et $t_{max}$. D'autres paramètres, appelés ci-après paramètres temps-dépendants, sont en revanche susceptibles d'évoluer dans le temps, par exemples les paramètres $k_{b1}$, $k_{b2}$, $k_{b3}$, $EGP_0$, $F_{01}$ et $F_R$. Du fait de cette variabilité de certains paramètres du système, il est en pratique nécessaire de ré-étalonner ou re-calibrer régulièrement le modèle en cours d'utilisation, par exemple toutes les 1 à 20 minutes, par exemple toutes les 5 minutes, pour s'assurer que les prédictions du modèle restent pertinentes. Cette mise à jour du modèle, aussi appelée personnalisation du modèle, doit pouvoir être réalisée de façon automatique par le système de la figure 1, c'est-à-dire sans qu'il soit nécessaire de mesurer physiquement les paramètres temps-dépendants du système sur le patient, puis de les transmettre à l'unité de traitement et de contrôle 105.

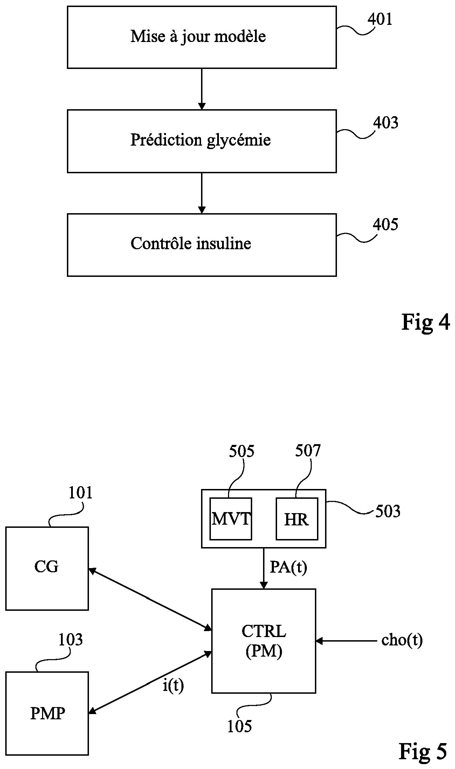

La figure 4 est un diagramme illustrant un exemple d'un procédé automatisé de régulation de glycémie mis en oeuvre par le système de la figure 1.

Ce procédé comprend une étape 401 de re-calibration ou de mise à jour du modèle, pouvant par exemple être répétée à intervalles réguliers, par exemple toutes les 1 à 20 minutes. Lors de cette étape, l'unité de traitement et de contrôle 105 met en oeuvre un procédé de ré-estimation des paramètres temps-dépendants du modèle en tenant compte des données d'insuline effectivement injectée par le dispositif 103 et des données de glycémie réelle mesurée par le capteur 101 pendant une période d'observation passée de durée $\Delta T$, par exemple une période de 1 à 10 heures précédant l'étape de calibration. Plus particulièrement, lors de l'étape de calibration, l'unité de traitement et de contrôle 105 simule le comportement du patient sur la période d'observation passée à partir du modèle physiologique (en tenant compte des éventuelles ingestions de glucose et injections d'insuline pendant cette période), et compare la courbe de glycémie $G(t)$ estimée par le modèle à la courbe de glycémie réelle $G^r(t)$ mesurée par le capteur pendant cette même période. L'unité de traitement et de contrôle 105 recherche alors, pour les paramètres temps-dépendants du modèle, un jeu de valeurs conduisant à minimiser une grandeur représentative de l'erreur entre la courbe de glycémie estimée par le modèle et la courbe de glycémie réelle pendant la période d'observation.

A titre d'exemple, l'unité de traitement et de contrôle recherche un jeu de paramètres conduisant à minimiser un indicateur m représentatif de l'écart quadratique moyen entre la glycémie estimée $G(t)$ et la glycémie réelle $G^r(t)$, par exemple défini comme suit :

$$m = \frac{1}{\Delta T} \sum_{t=t_0}^{t_0+\Delta T} |G^r(t) - G(t)|^2 \qquad (\text{eq15})$$

où t est la variable temps discrétisée, $t_0$ correspond à l'instant de début de la phase d'observation passé, $t_0+\Delta T$ correspond à l'instant de fin de la phase d'observation passée (correspondant par exemple à l'instant de début de l'étape de calibration du modèle), $G^r$ est la courbe d'évolution temporelle de la glycémie réelle mesurée par le capteur 101 pendant la période [t0, $t_0+\Delta T$], et G est la courbe de glycémie estimée à partir du modèle pendant la période [$t_0$, $t_0+\Delta T$]. L'algorithme de recherche de paramètres optimaux utilisé lors de cette étape n'est pas détaillé dans la présente demande, les modes de réalisation décrits étant compatibles avec les algorithmes usuels utilisés dans des domaines variés pour résoudre des problèmes d'optimisation de paramètres par minimisation d'une fonction de coût.

On notera que lors de l'étape 401, outre les paramètres temps-dépendants du modèle, l'unité de traitement et de contrôle 105 définit un vecteur [INIT] d'états initiaux (états à l'instant $t_0-\Delta T$) des variables d'état du modèle, pour pouvoir simuler le comportement du patient à partir du modèle. Pour définir les états initiaux des variables d'état du modèle, une première possibilité consiste à faire l'hypothèse que, dans la période précédant la période d'observation [$t_0-\Delta T$, $t_0$] sur laquelle est basée la calibration du modèle, le patient se trouvait dans un état stationnaire, avec un débit d'insuline injectée constant, et une prise alimentaire de glucose nulle. Sous cette hypothèse, toutes les dérivées du système d'équations différentielles peuvent être considérées comme nulles à l'instant initial $t_0-\Delta T$. Les valeurs à l'instant $t_0-\Delta T$ des variables d'état du système peuvent alors être calculées analytiquement. Pour améliorer l'initialisation, une autre possibilité consiste à faire les mêmes hypothèses que précédemment, mais en ajoutant la contrainte que la glycémie estimée à l'instant $t_0-\Delta T$ soit égale à la glycémie réelle mesurée par le capteur. Pour améliorer encore l'initialisation, une autre possibilité est de considérer les états initiaux des variables d'état du modèle comme des variables aléatoires, au même titre que les paramètres temps-dépendants du modèle. Les états initiaux des variables d'état sont alors déterminés de la même façon que les paramètres temps-dépendants du modèle, c'est-à-dire que l'unité de traitement et de contrôle 105 recherche un jeu de valeurs d'états initiaux [INIT] conduisant à minimiser une grandeur représentative de l'erreur entre la courbe de glycémie estimée par le modèle et la courbe de glycémie réelle pendant la période d'observation passée.

Le procédé de la figure 4 comprend en outre, après l'étape 401, une étape 403 de prédiction, par l'unité de traitement et de contrôle 105, de l'évolution temporelle de la glycémie du patient sur une période à venir [t0, $t_0+T_{pred}$] de durée $T_{pred}$, par exemple comprise entre 1 et 10 heures, à partir du modèle physiologique mis à jour à l'étape 401 et en tenant compte de l'historique d'insuline injectée au patient et de l'historique de glucose ingéré par le patient.

Le procédé de la figure 4 comprend de plus, après l'étape 403, une étape 405 de détermination, par l'unité de traitement et de contrôle 105, en tenant compte de la courbe de glycémie future prédite à l'étape 403, des doses d'insuline à injecter au patient pendant une période à venir [t0, $t_0+T_{pred}$]. A l'issue de cette étape, l'unité de traitement et de contrôle 105 peut programmer le dispositif d'injection 103 pour administrer les doses déterminées pendant la période de prédiction [t0, $t_0+T_{pred}$] à venir.

Les étapes 403 de prédiction de la glycémie et 405 de détermination des doses futures d'insuline à administrer peuvent par exemple être réitérées à chaque mise à jour du modèle physiologique (c'est-à-dire après chaque itération de l'étape 401), à chaque nouvelle ingestion de glucose signalée par le patient, et/ou à chaque nouvelle administration d'une dose d'insuline par le dispositif d'injection 103.

Dans un système de régulation du type décrit en relation avec les figures 1 à 4, la fiabilité de la prédiction de l'évolution future de la glycémie est particulièrement importante pour déterminer correctement les doses d'insuline à administrer au patient, et par conséquent pour réguler correctement la glycémie du patient.

Plus généralement, dans de nombreuses applications de contrôle automatisé de glycémie, la prédiction de l'évolution future de la glycémie du patient joue un rôle important pour permettre le maintien de la glycémie du patient dans une plage souhaitée (correspondant par exemple à une plage de normoglycémie) .

Une limitation des modèles physiologiques connus pour la prédiction de l'évolution future de la glycémie d'un patient est qu'ils ne tiennent pas compte de l'activité physique du patient, qui a pourtant des effets significatifs sur sa glycémie. Ainsi, il arrive, notamment pendant et/ou autour des périodes d'activité physique du patient, que les prédictions réalisées par ces modèles ne soient pas fiables. Ceci peut conduire à des erreurs lors du contrôle ou lors de régulation de la glycémie du patient, ce qui peut entraîner des risques pour le patient.

Selon un aspect des modes de réalisation décrits, on prévoit un système dans lequel la prédiction de l'évolution future de la glycémie du patient est réalisée au moyen d'un modèle physiologique prenant pour entrées non seulement la quantité d'insuline injectée au patient et la quantité de glucose ingéré par le patient, mais aussi une donnée représentative de l'activité physique du patient.

La figure 5 représente de façon schématique, sous forme de blocs, un exemple d'un mode de réalisation d'un système automatisé de prédiction de la glycémie d'un patient.

Le système de la figure 5 comprend les mêmes éléments que le système de la figure 1, agencés sensiblement de la même manière, et diffère du système de la figure 1 essentiellement en ce que, en fonctionnement, l'unité de traitement et de contrôle 105 est adaptée à recevoir non seulement des données cho(t) représentatives de l'évolution, en fonction du temps, de la quantité de glucose ingérée par le patient et des données i(t) représentatives de l'évolution, en fonction du temps, de la quantité d'insuline injectée au patient, mais aussi des données PA(t) représentatives de l'évolution, en fonction du temps, de l'activité physique du patient.

Comme dans l'exemple de la figure 1, les données de glucose ingéré cho(t) peuvent être saisies par le patient par l'intermédiaire d'une interface utilisateur non détaillée.

Les données d'insuline injectée i(t) peuvent également être saisies par le patient par l'intermédiaire d'une interface utilisateur. A titre de variante, les données d'insuline injectées i(t) sont directement communiquées à l'unité de traitement et de contrôle 105 par le dispositif d'injection d'insuline 103. Dans le cas où le dispositif d'injection d'insuline 103 est commandé exclusivement par l'unité de traitement et de contrôle 105, les données d'insuline injectée i(t) sont connues de l'unité de traitement et de contrôle 105 et n'ont donc pas besoin de lui être transmises.

Dans l'exemple de la figure 5, le système comprend un dispositif 503 adapté à mesurer l'activité physique du patient. Le dispositif 503 est relié à l'unité de traitement et de contrôle 105, par exemple par liaison filaire ou par liaison radio (sans fil), et communique à l'unité de traitement et de contrôle 105 les données d'activité physique PA(t) du patient. Le dispositif 503 est par exemple un dispositif mobile porté par le patient tout au long de la journée et/ou de la nuit. A titre d'exemple, le dispositif 503 comprend au moins un capteur de mouvements 505 (MVT), par exemple un accéléromètre. Le dispositif 503 peut en outre comprendre un capteur 507 (HR) du rythme cardiaque du patient.

Dans le mode de réalisation de la figure 5, l'unité de traitement et de contrôle 105 est configurée pour, à partir d'un modèle physiologique tenant compte de l'historique d'insuline injectée, de l'historique de glucose ingéré, et de l'historique d'activité physique du patient, déterminer une courbe représentative de l'évolution attendue de la glycémie du patient en fonction du temps, sur une période à venir. L'unité de traitement et de contrôle 105 peut en outre être configurée pour, en tenant compte de la courbe de glycémie prédite, mettre en oeuvre une action de contrôle ou de régulation de la glycémie du patient, dans le but de maintenir la glycémie du patient dans une plage souhaitée, par exemple de façon similaire à ce qui a été décrit en relation avec les figures 1 à 4.

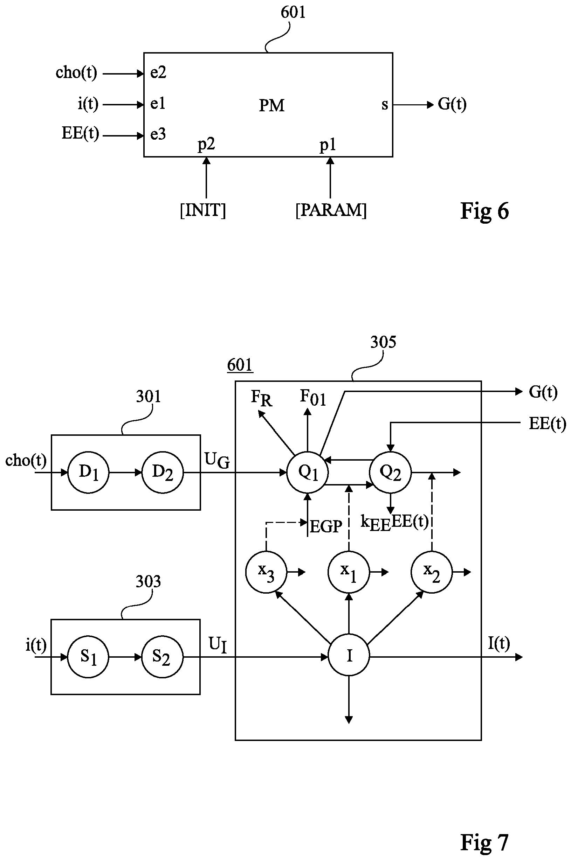

La figure 6 est une représentation simplifiée d'un modèle physiologique 601 (PM) utilisé dans le système de la figure 5 pour prédire l'évolution future de la glycémie du patient. Sur la figure 6, le modèle est représenté sous la forme d'un bloc de traitement comportant :

une entrée e1 sur laquelle est appliquée un signal i(t) représentatif de l'évolution, en fonction du temps t, de la quantité d'insuline injectée au patient ;
une entrée e2 sur laquelle est appliquée un signal cho(t) représentatif de l'évolution, en fonction du temps t, de la quantité de glucose ingérée par le patient ;
une entrée e3 sur laquelle est appliquée un signal EE(t) représentatif de l'évolution, en fonction du temps t, de la dépense énergétique du patient ; et
une sortie s fournissant un signal G(t) représentatif de l'évolution, en fonction du temps t, de la glycémie du patient.

A titre d'exemple, le signal EE(t) est représentatif de la quantité d'énergie dépensée à l'instant t par le patient pour permettre à son organisme de fonctionner correctement, d'assurer toutes ses fonctions physiques, et, le cas échéant, de réaliser un effort physique.

La variable d'entrée EE(t) du modèle peut être calculée à partir des signaux de sortie du capteur de mouvements 505 et du capteur de rythme cardiaque 507 du système de la figure 5. A titre d'exemple, le capteur de mouvements 505 fournit un signal $S_{CPM}$ représentatif des mouvements effectués par le patient, par exemple représentatif d'un nombre de mouvements par minute effectués par le patient, par exemple représentatif du nombre de pas par minute effectués par le patient, et le capteur de rythme cardiaque 507 fournit un signal $S_{HR}$ représentatif du rythme cardiaque (nombre de pulsations par minute) du patient. Plus particulièrement, dans cet exemple, le signal $S_{HR}$ est égal à la différence entre le rythme cardiaque (nombre de pulsations par minute) du patient à l'instant t et le rythme cardiaque au repos du patient (qui peut être une constante du système). La variable d'entrée EE(t) peut alors être calculée comme suit :

$$EE(t) = \begin{cases} \alpha_1 * S_{HR}(t) + \beta_1, & S_{HR} \geq S1 \\ \alpha_2 * S_{LC}(t) + \beta_2, & S_{HR} < S1 \ et \ S_{LC} < S2 \\ \alpha_3 * S_{LC}(t) + \beta_3, & S_{HR} < S1 \ et \ S_{LC} \geq S2 \end{cases} \qquad (eq16)$$

où le signal $S_{LC}$ est une combinaison linéaire des signaux $S_{HR}$ et $S_{CMP}$, telle que $S_{LC}(t) = \theta1*S_{CMP}(t)+\theta2*S_{HR}(t)$, et où les grandeurs $\alpha_1$, $\alpha_2$, $\alpha_3$, $\beta_1$, $\beta_2$, $\beta_3$, $\theta1$, $\theta2$, S1 et S2 sont des paramètres du système. Un exemple de procédé d'estimation de la dépense énergétique d'un individu, pouvant être utilisé pour calculer la variable d'entrée EE(t) du modèle, est décrit plus en détail dans l'article intitulé "An original piecewise model for computing energy expenditure from accelerometer and heart rate signals" de H.M. Romero-Ugalde et al. (Physiological Measurement, vol. 38, no. 8, p. 1599, 2017).

Le modèle 601 est un modèle physiologique, c'est-à-dire un modèle mathématique constitué par un système d'équations modélisant les différents mécanismes physiologiques s'opérant dans le corps du patient et ayant un effet sur sa glycémie. Le modèle 601 est par exemple un modèle compartimental comportant, outre les variables d'entrée i(t), cho(t) et EE(t) et la variable de sortie G(t), une pluralité de variables d'état correspondant à des variables physiologiques du patient, évoluant en fonction du temps. De façon similaire à ce qui a été décrit en relation avec les figures 1 à 4, l'évolution temporelle des variables d'état et de la variable de sortie G(t) est régie par un système d'équations différentielles comportant une pluralité de paramètres représentés sur la figure 6 par un vecteur [PARAM] appliqué sur une entrée p1 du bloc 601. La réponse du modèle physiologique est en outre conditionnée par les états initiaux ou valeurs initiales affectés aux variables d'état, représentés sur la figure 2 par un vecteur [INIT] appliqué sur une entrée p2 du bloc 601.

Selon un aspect d'un mode de réalisation, le modèle physiologique 601 comprend au moins une équation comportant un terme $-(k_{EE}*EE(t))$, où $k_{EE}$ est un paramètre du modèle, par exemple un paramètre constant ou un paramètre temps-dépendant ré-estimé à chaque mise à jour du modèle (étape 401 du procédé de la figure 4). Le terme $-(k_{EE}*EE(t))$ correspond à un puits de glucose représentant la consommation de glucose par les muscles, notamment durant une activité physique. Le terme $-(k_{EE}*EE(t))$ est par exemple pris en compte pour calculer la dérivée temporelle d'une variable du modèle représentative d'une masse de glucose dans un compartiment.

A titre d'exemple, le modèle physiologique 601 utilisé dans le système de la figure 5 est un modèle basé sur le modèle de Hovorka, adapté pour tenir compte de la dépense énergétique EE(t) du patient.

La figure 7 est un diagramme représentant plus en détail un exemple du modèle physiologique 601 utilisé dans le système de la figure 5 pour prédire l'évolution future de la glycémie du patient.

Le modèle physiologique de la figure 7 est similaire au modèle physiologique de la figure 3, et diffère du modèle de la figure 3 essentiellement en ce que, dans le modèle de la figure 7, le sous-modèle 305, décrivant la régulation du glucose par le corps du patient, prend pour entrées non seulement les taux d'absorption $U_G$ du glucose et $U_I$ de l'insuline, mais aussi la dépense énergétique EE(t) du patient. Plus particulièrement, dans l'exemple de la figure 7, l'évolution temporelle de la variable d'état Q2 dépend du terme $-(k_{EE}*EE(t))$. Dans cet exemple, le terme $k_{EE}*EE(t)$, correspondant à une quantité de glucose consommée par les muscles en fonction de la dépense énergétique EE(t), est soustrait à la quantité de glucose Q2 du modèle.

Le système d'équations régissant le modèle de la figure 7 est par exemple identique au système décrit en relation avec la figure 3, à la différence près que, dans le système de la figure 7, l'équation eq3 du système de la figure 3 est remplacée par l'équation eq3' suivante :

$$\frac{dQ_2}{dt} = x_1(t) \cdot Q_1(t) - [k_{12} + x_2(t)] \cdot Q_2(t) - k_{EE}.EE(t) \qquad (eq3')$$

De même que dans l'exemple de la figure 3, on a représenté sur la figure 7 une sortie délivrant un signal I(t) représentatif de l'évolution, en fonction du temps, de la variable I. Cette sortie est toutefois optionnelle.

A titre d'exemple, le système de la figure 5 peut être configuré pour mettre en oeuvre un procédé automatisé de régulation de glycémie identique ou similaire au procédé décrit en relation avec la figure 4, basé sur le modèle physiologique 601 décrit en relation avec les figures 6 et 7 en remplacement du modèle 201 décrit en relation avec les figures 2 et 3.

Dans ce cas, lors de l'étape 401 de re-calibration ou mise à jour du modèle, l'unité de traitement et de contrôle 105 tient compte, pour ré-estimer les paramètres temps-dépendants du modèle, non seulement des données d'insuline effectivement injectée par le dispositif 103 et des données de glycémie réelle mesurée par le capteur 101 pendant la période d'observation passée ΔT, mais aussi des données de dépense énergétique du patient sur la période d'observation passée ΔT, déterminées au moyen du dispositif de mesure d'activité physique 503.

Lors de l'étape 403 l'unité de traitement et de contrôle 105 prédit l'évolution temporelle de la glycémie du patient sur une période à venir [t0, $t_0+T_{pred}$] de durée $T_{pred}$, à partir du modèle physiologique mis à jour à l'étape 401 et en tenant compte de l'historique d'insuline injectée au patient, de l'historique de glucose ingéré par le patient, et de l'historique de dépense énergétique du patient.

Lors de l'étape 405, l'unité de traitement et de contrôle 105 détermine, en tenant compte de la courbe de glycémie future prédite à l'étape 403, des doses d'insuline à injecter au patient pendant la période à venir [t0, $t_0+T_{pred}$]. A l'issue de cette étape, l'unité de traitement et de contrôle 105 peut en outre programmer le dispositif d'injection 103 pour administrer les doses déterminées pendant la période de prédiction [t0, $t_0+T_{pred}$] à venir.

Les étapes 403 de prédiction de la glycémie et 405 de détermination des doses futures d'insuline à administrer peuvent par exemple être réitérées à chaque mise à jour du modèle physiologique (c'est-à-dire après chaque itération de l'étape 401), à chaque nouvelle ingestion de glucose signalée par le patient, à chaque nouvelle administration d'une dose d'insuline par le dispositif d'injection 103, et/ou à chaque fois qu'une activité physique significative du patient est détectée.

Un avantage du système décrit en relation avec les figures 5, 6 et 7 est qu'il permet de réaliser des prédictions de glycémie plus fiables que les systèmes basés sur des modèles physiologiques ne prenant pas en compte l'activité physique du patient, notamment pendant et autour des périodes d'activité physique.

Des modes de réalisation particuliers ont été décrits. Diverses variantes et modifications apparaîtront à l'homme de l'art. En particulier, les modes de réalisation décrits ne se limitent pas aux exemples décrits d'utilisation du système de prédiction de glycémie de la figure 5. Plus généralement, le système de prédiction proposé, basé sur l'utilisation d'un modèle physiologique tenant compte de l'activité physique du patient, peut être utilisé pour toute application pouvant tirer profit d'une prédiction fiable de l'évolution future de la glycémie d'un patient.

De plus, les modes de réalisation décrits ne se limitent pas à l'exemple décrit ci-dessus dans lequel la variable d'entrée EE(t) du modèle est calculée en tenant compte d'un signal de sortie d'un capteur des mouvements du patient et d'un signal de sortie d'un capteur du rythme cardiaque du patient. A titre de variante, la variable d'entrée EE(t) peut être calculée en tenant compte uniquement du signal de sortie du capteur de mouvements ou uniquement du signal de sortie du capteur de rythme cardiaque. Plus généralement, la variable d'entrée EE(t) peut être constituée par tout signal ou combinaison de signaux représentatifs de l'activité physique du patient. A titre d'exemple, la variable d'entrée EE(t) peut être une combinaison d'un signal mesuré au moyen d'un capteur quelconque, par exemple le capteur de mouvements 505 et/ou le capteur de rythme cardiaque 507 de la figure 5, et d'un signal d'intensité d'activité physique déclaré par le patient au moyen d'une interface utilisateur (non détaillée). A titre de variante, la variable d'entrée EE(t) peut être calculée uniquement à partir d'un signal d'intensité d'activité déclaré par le patient.

Par ailleurs, les modes de réalisation décrits ne se limitent pas au cas particulier décrit en relation avec la figure 7 dans lequel le modèle physiologique est une adaptation du modèle de Hovorka. Plus généralement, les modes de réalisation décrits sont compatibles avec tout autre modèle physiologique décrivant l'assimilation de l'insuline par le corps d'un patient et son effet sur la glycémie du patient, et tenant compte de la dépense énergétique du patient, et, plus particulièrement d'une consommation de glucose par les muscles liée à la dépense énergétique du patient (le terme $-(k_{EE}*EE(t))$ décrit ci-dessus).

**Revendications**

1.  Système automatisé de contrôle de la glycémie d'un patient, comportant une unité de traitement et de contrôle (105) configurée pour prédire l'évolution future de la glycémie du patient à partir d'un modèle physiologique (601), dans lequel le modèle physiologique (601) comporte un système d'équations différentielles décrivant l'évolution d'une pluralité de variables d'état (D1, D2, S1, S2, Q1, Q2, x1, x2, x3, I) en fonction du temps, et dans lequel au moins l'une des équations du système prend pour entrée une variable EE(t) représentative de l'évolution, en fonction du temps, de la dépense énergétique du patient,

dans lequel ladite au moins une équation est définie comme suit :

$$\frac{dQ_2}{dt} = x_1(t) \cdot Q_1(t) - [k_{12} + x_2(t)] \cdot Q_2(t) - k_{EE}.EE(t)$$

où $Q_1$ et $Q_2$ sont des variables d'état correspondant respectivement à des masses de glucose dans des premier et deuxième compartiments, où $x_1$ et $x_2$ sont des variables d'état représentant chacune une action de l'insuline sur la cinétique du glucose, et où $k_{12}$ et $k_{EE}$ sont des paramètres du modèle physiologique (601), le terme $-(k_{EE}*EE(t))$ étant représentatif d'une consommation de glucose par les muscles.

2. Système selon la revendication 1, comprenant un dispositif (503) de mesure d'une activité physique du patient, la variable d'entrée EE(t) du modèle (601) étant calculée par l'unité de traitement et de contrôle (105) à partir de données de sortie PA(t) du dispositif de mesure (503).

3. Système selon la revendication 2, dans lequel le dispositif de mesure (503) comprend un capteur (505) de mouvements du patient.

4. Système selon la revendication 3, dans lequel le dispositif de mesure (503) comprend en outre un capteur (507) du rythme cardiaque du patient.

5. Système selon la revendication 4, dans lequel le capteur de mouvements (505) fournit un signal $S_{CPM}$ représentatif de mouvements effectués par le patient, et le capteur de rythme cardiaque (507) fournit un signal $S_{HR}$ représentatif du rythme cardiaque du patient, et dans lequel la variable d'entrée EE(t) du modèle (601) est calculée comme suit par l'unité de traitement et de contrôle (105) :

$$EE(t) = \begin{cases} \alpha_1 * S_{HR}(t) + \beta_1, & S_{HR} \geq S1 \\ \alpha_2 * S_{LC}(t) + \beta_2, & S_{HR} < S1 \ et \ S_{LC} < S2 \\ \alpha_3 * S_{LC}(t) + \beta_3, & S_{HR} < S1 \ et \ S_{LC} \geq S2 \end{cases}$$

où le signal $S_{LC}$ est une combinaison linéaire des signaux $S_{HR}$ et $S_{CMP}$, et où les grandeurs $\alpha_1$, $\alpha_2$, $\alpha_3$, $\beta_1$, $\beta_2$, $\beta_3$, S1 et S2 sont des paramètres du système.

6. Système selon l'une quelconque des revendications 1 à 5, dans lequel le modèle physiologique (601) prend en outre pour entrées une variable i(t) représentative de l'évolution, en fonction du temps, de la quantité d'insuline injectée au patient, et une variable cho(t) représentative de l'évolution, en fonction du temps, de la quantité de glucose ingérée par le patient.

7. Système selon l'une quelconque des revendications 1 à 6, comportant en outre un capteur de glycémie (101).

8. Système selon la revendication 7, dans lequel l'unité de traitement et de contrôle (105) est adaptée à mettre en oeuvre une étape de calibration automatique du modèle physiologique comprenant une étape d'estimation d'un jeu de paramètres ([PARAM]) du système d'équations différentielles par minimisation d'une grandeur m représentative de l'erreur, pendant une période d'observation passée, entre la glycémie estimée à partir du modèle physiologique et la glycémie mesurée par le capteur (101).

9. Système selon la revendication 8, dans lequel ladite grandeur est définie comme suit :

$$m = \frac{1}{\Delta T} \sum_{t=t_0}^{t_0+\Delta T} |G^r(t) - G(t)|^2$$

où t est une variable temps discrétisée, $t_0$ est l'instant de début de la période d'observation passée, et $t_0+\Delta T$ est l'instant de fin de la période d'observation passée.

**10.** Système selon l'une quelconque des revendications 1 à 9, comportant en outre un dispositif d'injection d'insuline (103), dans lequel l'unité de traitement et de contrôle (105) est adaptée à commander le dispositif d'injection d'insuline en tenant compte de la prédiction de l'évolution future de la glycémie du patient réalisée à partir du modèle physiologique (601).

**Patentansprüche**

**1.** Ein automatisches System zum Kontrollieren bzw. Steuern des Blutglukose- bzw. Blutzuckerspiegels eines Patienten, das eine Verarbeitungs- und Steuereinheit (105) aufweist, die konfiguriert ist, den zukünftigen Trend des Blutglukosespiegels des Patienten auf Grundlage eines physiologischen Modells (601) vorherzusagen, wobei das physiologische Modell (601) ein Differentialgleichungssystem aufweist, das die zeitliche Variation einer Vielzahl von Zustandsvariablen (D1, D2, S1, S2, Q1, Q2, x1, x2, x3, I) beschreibt, und wobei mindestens eine der Gleichungen des Systems als eine Eingang eine Variable EE(t) nimmt, die für die zeitliche Variation des Energieverbrauchs des Patienten charakteristisch ist,

wobei die mindestens eine Gleichung wie folgt definiert ist:

$$\frac{dQ_2}{dt} = x_1(t) \cdot Q_1(t) - [k_{12} + x_2(t)] \cdot Q_2(t) - k_{EE}.EE(t)$$

wobei $Q_1$ und $Q_2$ Zustandsvariablen sind, die jeweils Glukosemengen bzw. -massen in ersten und zweiten Kompartimenten entsprechen, wobei $x_1$ und $x_2$ Zustandsvariablen sind, wobei jede für eine Wirkung von Insulin auf die Kinetik von Glukose steht, wobei $k_{12}$ und $k_{EE}$ Parameter des physiologischen Modells (601) sind, wobei der Term -($k_{EE}$ *EE(t)) für einen Glukoseverbrauch durch die Muskeln charakteristisch ist.

**2.** System nach Anspruch 1, die eine Einrichtung (503) zum Messen einer körperlichen Aktivität des Patienten aufweist, wobei die Eingangsvariable EE(t) des Modells (601) von der Verarbeitungs- und Steuereinheit (105) auf Grundlage der Ausgangsdaten PA(t) der Messeinrichtung (503) berechnet wird.

**3.** System nach Anspruch 2, wobei die Messeinrichtung (503) einen Sensor (505) für die Bewegungen des Patienten aufweist.

**4.** System nach Anspruch 3, wobei die Messeinrichtung (503) ferner einen Sensor (507) für die Herzfrequenz des Patienten aufweist.

**5.** System nach Anspruch 4, wobei der Bewegungssensor (505) ein Signal $S_{CPM}$ liefert, das für die vom Patienten ausgeführte Bewegungen charakteristisch ist, und wobei der Herzfrequenzsensor (507) ein Signal $S_{HR}$ liefert, das für die Herzfrequenz des Patienten charakteristisch ist, und wobei die Eingangsvariable EE(t) des Modells (601) von der Verarbeitungs- und Steuereinheit (105) wie folgt berechnet wird:

$$EE(t) = \begin{cases} \alpha_1 * S_{HR}(t) + \beta_1, & S_{HR} \geq S1 \\ \alpha_2 * S_{LC}(t) + \beta_2, & S_{HR} < S1 \: et \: S_{LC} < S2 \\ \alpha_3 * S_{LC}(t) + \beta_3, & S_{HR} < S1 \: et \: S_{LC} \geq S2 \end{cases}$$

wobei das Signal $S_{LC}$ eine lineare Kombination der Signale $S_{HR}$ und $S_{CPM}$ ist und wobei die Größen $\alpha_1$, $\alpha_2$, $\alpha_3$, $\beta_1$, $\beta_2$, $\beta_3$, S1 und S2 Parameter des Systems sind.

**6.** System nach einem der Ansprüche 1 bis 5, wobei das physiologische Modell (601) ferner als Eingänge eine Variable i(t) nimmt, die für die zeitliche Variation der dem Patienten injizierten Insulinmenge charakteristisch ist, und eine Variable cho(t), die für die zeitliche Variation der vom Patienten eingenommenen Kohlenhydratmenge charakteristisch ist.

**7.** System nach einem der Ansprüche 1 bis 6, das ferner einen Blutglukose- bzw. Blutzuckersensor (101) aufweist.

8. System nach Anspruch 7, wobei die Verarbeitungs- und Steuereinheit (105) in der Lage ist, einen automatischen Kalibrierungsschritt des physiologischen Modells zu implementieren, der einen Schritt zur Schätzung eines Satzes von Parametern ([PARAM]) des Differentialgleichungssystems durch Minimierung einer Größe m aufweist, die für den Fehler, und zwar während eines vergangenen Beobachtungszeitraums, zwischen der auf Grundlage des physiologischen Modells geschätzten Blutglukose und der vom Sensor (101) gemessenen Blutglukose charakteristisch ist.

9. System nach Anspruch 8, wobei die Größe wie folgt definiert ist:

$$m = \frac{1}{\Delta T} \sum_{t=t_0}^{t_0+\Delta T} |G^r(t) - G(t)|^2$$

wobei t eine diskretisierte Zeitvariable ist, wobei $t_0$ der Zeitpunkt des Beginns des vergangenen Beobachtungszeitraums ist und wobei $t_0+\Delta T$ der Zeitpunkt des Endes des vergangenen Beobachtungszeitraums ist.

10. System nach einem der Ansprüche 1 bis 9, wobei das System ferner eine Insulininjektionseinrichtung (103) aufweist, wobei die Verarbeitungs- und Steuereinheit (105) in der Lage ist, die Insulininjektionseinrichtung zu steuern, und zwar unter Berücksichtigung der Vorhersage des zukünftigen Trends der Blutglukose des Patienten, die auf Grundlage des physiologischen Modells (601) gemacht wurde.

**Claims**

1. An automated system for controlling a patient's blood glucose, comprising a processing and control unit (105) configured to predict the future trend of the patient's blood glucose based on a physiological model (601), wherein the physiological model (601) comprises a differential equation system describing the time variation of a plurality of state variables (D1, D2, S1, S2, Q1, Q2, x1, x2, x3, I), and wherein at least one of the equations of the system takes as an input a variable EE(t) representative of the time variation of the patient's energy expenditure,

wherein said at least one equation is defined as follows:

$$\frac{dQ_2}{dt} = x_1(t) \cdot Q_1(t) - [k_{12} + x_2(t)] \cdot Q_2(t) - k_{EE}.EE(t)$$

where $Q_1$ and $Q_2$ are state variables respectively corresponding to glucose masses in first and second compartments, where $x_1$ and $x_2$ are state variables, each standing for an action of insulin on the kinetics of glucose, where $k_{12}$ and $k_{EE}$ are parameters of the physiological model (601), term $-(k_{EE}*EE(t))$ being representative of a glucose consumption by the muscles.

2. System according to claim 1, comprising a device (503) for measuring a physical activity of the patient, the input variable EE(t) of the model (601) being calculated by the processing and control unit (105) based on the output data PA(t) of the measurement device (503).

3. System according to claim 2, wherein the measurement device (503) comprises a sensor (505) of the patient's motions.

4. System according to claim 3, wherein the measurement device (503) further comprises a sensor (507) of the patient's heart rate.

5. System according to claim 4, wherein the motion sensor (505) delivers a signal $S_{CPM}$ representative of motions performed by the patient, and the heart rate sensor (507) delivers a signal $S_{HR}$ representative of the patient's heart rate, and wherein the input variable EE(t) of the model (601) is calculated as follows by the processing and control unit (105):

$$EE(t) = \begin{cases} \alpha_1 * S_{HR}(t) + \beta_1, & S_{HR} \geq S1 \\ \alpha_2 * S_{LC}(t) + \beta_2, & S_{HR} < S1 \ et \ S_{LC} < S2 \\ \alpha_3 * S_{LC}(t) + \beta_3, & S_{HR} < S1 \ et \ S_{LC} \geq S2 \end{cases}$$

where signal $S_{LC}$ is a linear combination of signals $S_{HR}$ and $S_{CMP}$, and where quantities $\alpha_1$, $\alpha_2$, $\alpha_3$, $\beta_1$, $\beta_2$, $\beta_3$, S1, and S2 are parameters of the system.

6. System according to any of claims 1 to 5, wherein the physiological model (601) further takes as inputs a variable i(t) representative of the time variation of the quantity of insulin injected to the patient, and a variable cho(t) representative of the time variation of the quantity of carbohydrates ingested by the patient.

7. System according to any of claims 1 to 6, further comprising a blood glucose sensor (101).

8. System according to claim 7, wherein the processing and control unit (105) is capable of implementing a step of automatic calibration of the physiological model comprising a step of estimation of a set of parameters ([PARAM]) of the differential equation system by minimization of a quantity m representative of the error, during a past observation period, between the blood glucose estimated based on the physiological model and the blood glucose measured by the sensor (101).

9. System according to claim 8, wherein said quantity is defined as follows:

$$m = \frac{1}{\Delta T} \sum_{t=t_0}^{t_0 + \Delta T} |G^r(t) - G(t)|^2$$

where t is a discretized time variable, $t_0$ is the time of beginning of the past observation period, and $t_0 + \Delta T$ is the time of end of the past observation period.

10. System according to any of claims 1 to 9, further comprising an insulin injection device (103), wherein the processing and control unit (105) is capable of controlling the insulin injection device, taking into account the prediction of the future trend of the patient's blood glucose made based on the physiological model (601).

Fig 1

Fig 2

Fig 3

**Fig 4**

**Fig 5**

**Fig 6**

**Fig 7**

## RÉFÉRENCES CITÉES DANS LA DESCRIPTION

**Documents brevets cités dans la description**

- FR 1856016
- WO 2018055283 A
- WO 2018055284 A
- FR 1756960

**Littérature non-brevet citée dans la description**

- **ROMAN HOVORKA et al.** Nonlinear model prédictive control of glucose concentration in subjects with type 1 diabètes. *Physiol Meas.,* 2004, vol. 25, 905-920
- **ROMAN HOVORKA et al.** Partitioning glucose distribution/transport, disposal, and endogenous production during IVGTT. *Am J Physiol Endocrinol Metab,* 2002, vol. 282, E992-E1007
- **H.M. ROMERO-UGALDE et al.** An original piecewise model for computing energy expenditure from accelerometer and heart rate signals. *Physiological Measurement,* 2017, vol. 38 (8), 1599